# Europäisches Patentamt

# European Patent Office

# Office européen des brevets

(11) Veröffentlichungsnummer: **0 153 991**
**B1**

(12) **EUROPÄISCHE PATENTSCHRIFT**

(45) Veröffentlichungstag der Patentschrift:
25.11.87

(21) Anmeldenummer: **84111818.5**

(22) Anmeldetag: **03.10.84**

(51) Int. Cl.⁴: **A 61 M  16/00**

(54) Zuführungsstück für die Atemgase bei der Hochfrequenzbeatmung.

(30) Priorität: **20.01.84  DE 3401924**

(43) Veröffentlichungstag der Anmeldung:
**11.09.85 Patentblatt 85/37**

(45) Bekanntmachung des Hinweises auf die Patenterteilung:
**25.11.87 Patentblatt 87/48**

(84) Benannte Vertragsstaaten:
**AT DE FR GB IT NL SE**

(56) Entgegenhaltungen:
**DE-A-3 048 223**
**DE-B-1 491 659**
**DE-C-3 313 855**
**GB-A-2 118 442**

(73) Patentinhaber: **Drägerwerk Aktiengesellschaft,
Moislinger Allee 53- 55, D-2400 Lübeck 1 (DE)**

(72) Erfinder: **Schubert, Ernst W., Dipl.- Ing., Heinrich-
Mann- Ring 6, D-2400 Lübeck (DE)**
Erfinder: **Gebhardt, Peter, Dipl.- Phys.,
Gleiwitzstrasse 3, D-2406 Stockelsdorf (DE)**

## Beschreibung

Die Erfindung betrifft ein Zuführungsstück für die Atemgase bei der Hochfrequenzbeatmung nach dem Oberbegriff des Anspruches 1.

Ein Zuführungsstück wird in der DE-A-33 13 855, die keinen Stand der Technik gemäß Art. 54 EPÜ darstellt, beschrieben. Diese Druckschrift offenbart ein Beatmungssystem zur Hochfrequenzbeatmung eines Patienten beschreibt. In einer Ringleitung wird atembares Spülgas umgewälzt. Dabei ist ein Zuführungsstück mit einer Seite über einen Zuführungsstutzen und einen Abgabestutzen derart in diese Ringleitung eingeschaltet, daß der Spülgasstrom durch dessen Innenraum verläuft. Mit der anderen Seite ist dieser Innenraum mit einem Trachealtubus, also mit dem Patienten verbunden. Der Innenraum ist zwischen den beiden Seiten durch ein Strömungsleitstück strömungstechnisch in Teilräume aufgeteilt. Das Strömungsleitstück ist als doppelseitige Fangdüse ausgebildet, der an jeder Seite eine Injektordüse zugeordnet ist. Beide Injektordüsen ragen als am Ende gewinkelte Röhrchen durch die Wand des Führungsstückes in die Mitte des Teilraumes hinein. Sie sind an eine Atemgasquelle angeschlossen. Die auf der Seite der Ringleitung gelegene Injektordüse ist axial durch das Zuführungsstück in den Trachealtubus hineingerichtet. Sie wird in der Inspirationsphase als Jet-Düse mit hochfrequenten Impulsen betrieben und führt dem Patienten mit dem Jet-Atemgas zugleich aus der Ringleitung angesaugtes frisches Spülgas zu. Die auf der Seite des Trachealtubus gelegene Injektordüse ist axial durch das Zuführungsstück zur Ringleitung gerichtet. Sie wird als Ejektordüse betrieben und saugt das Atemgas aus der Lunge ab und drückt es in die Ringleitung hinein. Dies bekannte Zuführungsstück ist einteilig, daher nicht einfach herstellbar. Die in den freien Querschnitt hinein ragenden Injektordüsen sind störend bei der Reinigung, eine Anpassung des Zu- bzw. Abführungsstutzens z. B. durch Drehung an die günstigste Stellung zum Patienten ist nicht möglich.

In der DE-B-1 491 659 ist eine Atmungsvorrichtung der im ersten Teil des Anspruchs 1 genannten Art beschrieben, bei der zwischen einer Steuereinheit für das Atemgas und einem Tubusanschlußstück zu dem Patienten ein Zuführungsstück geschaltet ist. Während der Einatemphase wird über eine Injektordüse, deren Strahl in einen mit Fangdüsen versehenen Strömungsteil weist, zusätzliche Luft aus der Umgebungsatmosphäre angesaugt. Wird durch die Steuereinheit von der Einatemphase auf die Ausatemphase umgeschaltet, wird der Gasstrom aus der Injektordüse abgeschaltet, und eine der Injektordüse entgegengerichtete, auf der gegenüberliegenden Seite der Fangdüse befindliche Ejektordüse wird mit einem Gasstrom versorgt. Dadurch wird ein vom Tubusanschlußstück fortweisender, negativer Druckgradient erzeugt, welcher die im Luftweg des Patienten befindlichen Gase ansaugt und an die Atmosphäre abgibt.

Aufgabe der Erfindung ist ein Zuführungsstück für die Atemgase bei der Hochfrequenzbeatmung, das diese Schwierigkeiten nicht kennt, vor allem einfach reinigbar ohne Gefährdung der wichtigen Injektordüsen und in den Anschlüssen anpaßbar an die günstigste Stellung zum Patienten ist.

Diese Aufgabe wird bei einer gattungsgemäßen Einrichtung durch die kennzeichnenden Merkmale des Anspruchs 1 gelöst.

In weiterer Ausbildung der Erfindung sind die im spitzen Winkel zueinander stehenden Zuführungs- und Abgabestutzen und der Anschlußkonus jeweils rechtwinklig zu ihrem Rohrteil ausgebildet, erfolgt die Abdichtung zwischen den Teilen über Dichtungsringe, besitzt das Patiententeil dem Anschlußkonus entgegengesetzt einen verschließbaren Stutzen und besitzt der Anschlußkonus einen Anschluß für die Abnahme des Atemwegdruckes.

Die mit der Erfindung erzielten Vorteile bestehen insbesondere darin, daß in drei Einzelteile einfach zerlegbare Zuführungsstücke nicht nur gut herstellbar, sondern auch durch die Anordnung der Injektor- und der Ejektordüse in der Stirnwand der Rohrteile einfach reinigbar sind. Es besteht keine Gefahr, daß dabei möglicherweise die Düsen verbiegen oder sogar abbrechen, so daß sie ihre wichtige Funktion kaum prüfbar nicht mehr erfüllen können. Durch die Verdrehung der Teile zueinander ist eine Anpassung an die jeweilige Patientensituation vornehmbar.

Die Unteransprüche stellen zweckmäßige Ausbildungen des Gegenstandes des Hauptanspruches dar.

Ein Ausführungsbeispiel der Erfindung ist in der Zeichnung dargestellt und wird im folgenden beschrieben. Es zeigen

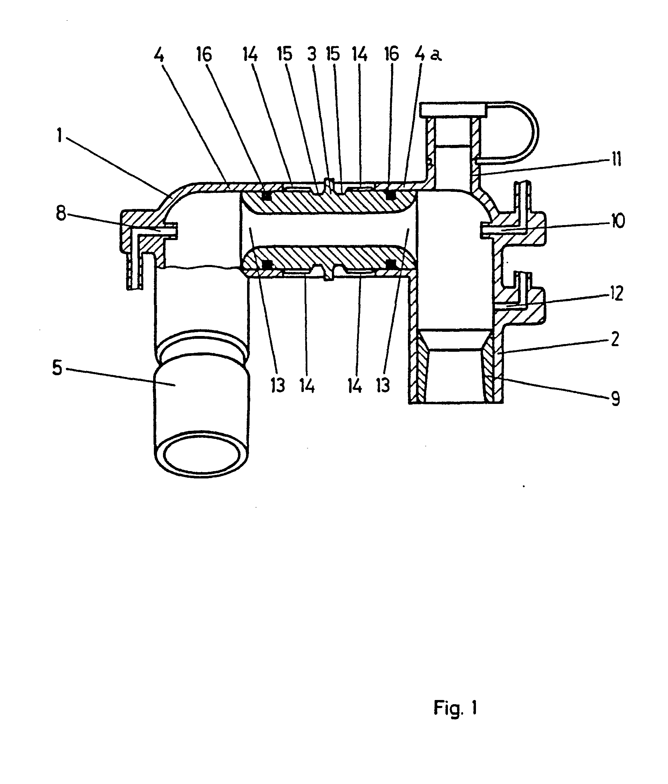

Fig. 1 ein Zuführungsstück im Längsschnitt

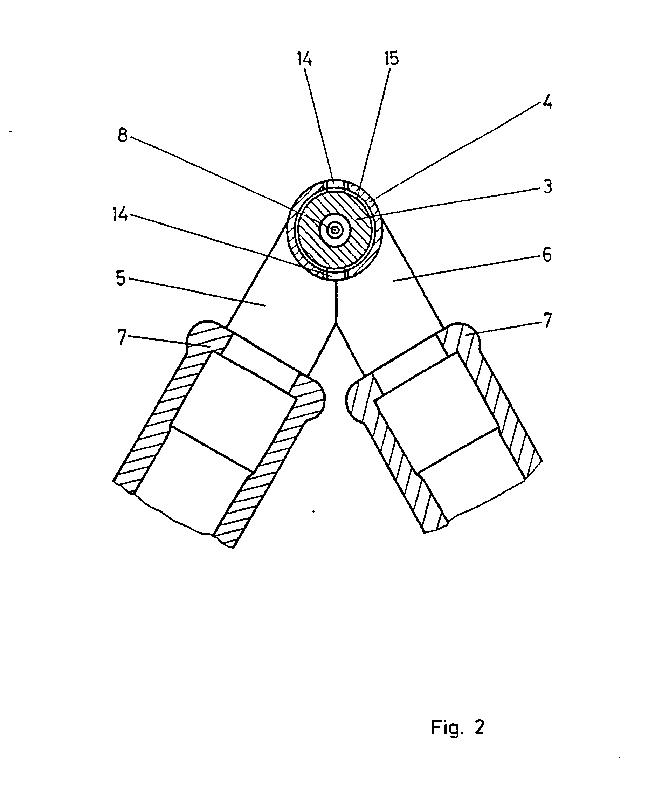

Fig. 2 im Querschnitt

Das Zuführungsstück für die Atemgase bei der Hochfrequenzbeatmung besteht aus einem Ringleitungsteil 1, einem Patiententeil 2 und einem beide Teile verbindenden Strömungsteil 3.

Das Ringleitungsteil 1 mit einem Rohrteil 4 besitzt rechtwinklig dazu in einem spitzen Winkel zueinander stehend einen Zuführungsstutzen 5, einen Abgabestutzen 6 zum Anschluß von Schläuchen 7 zum Einbau in das Ringleitungssystem für das Spülgas. Eine Injektordüse 8 in der Stirnwand zum Rohrteil 4 ist axial in das Strömungsteil 3 gerichtet, sie wird aus der Atemgasquelle versorgt.

Das Patiententeil 2 mit einem entgegengesetzten, sonst gleich ausgebildeten Rohrteil 4a hat rechtwinklig dazu einen genormten Anschlußkonus 9 für die Verbindung zum Patienten, z. B. über einen Endotrachealtubus. Eine Ejektordüse 10 in der Stirnwand zum Rohrteil 4 mit dem Anschluß an die Atemgasquelle ist axial in das Strömungsteil 3 gerichtet. Ein verschließbarer Stutzen 11 erlaubt die Einführung eines Absaugekatheters. Über einen Anschluß 12 kann der Atemwegdruck abgenommen werden.

Das Strömungsteil 3 erfüllt die Aufgabe der Fangdüse für die Injektordüse 8 und die Ejektordüse 10 und verbindet gleichzeitig, jedoch gegeneinander drehbar, das Ringleitungsteil 1 mit dem Patiententeil 2. Das Strömungsteil 3 ist symmetrisch mit gleichen Einströmöffnungen 13 ausgebildet.

Die Verbindung der drei Bauteile erfolgt über Schnapparme 14 in den Rohrteilen 4, 4a, die in außen umlaufenden Nuten 15 des Strömungsteils 3 eingreifen. Die Abdichtung zwischen den Teilen, die jedoch die Drehung gegeneinander erlauben, erfolgt über Dichtungsringe 16.

**Patentansprüche**

1. Zuführungsstück für die Atemgase bei der Hochfrequenzbeatmung mit einem Zuführungsstutzen (5) und einem Patiententeil (2), der einen Anschlußkonus (9) trägt, sowie mit einem dazwischen angeordneten Strömungsteil (3) mit Fangdüsen für eine in diese hineingerichtete Injektor-(8) und Ejektordüse (10), dadurch gekennzeichnet, daß ein Ringleitungsteil (1) mit dem Zuführungsstutzen (5) und einem in diesen mündenden Abgabestutzen (6) an einem Rohrteil (4) und das Patiententeil (2) an einem entsprechenden Rohrteil (4a) über Schnapparme (14) an diesen Rohrteilen (4, 4a) durch das Strömungsteil (3) drehbar miteinander verbunden und gehalten sin und daß die Injektordüse (8) und die Ejektordüse (10) jeweils in der Stirnwand des Rohrteils (4 bzw. 4a) angeordnet sind.

2. Zuführungsstück für die Atemgase nach Anspruch 1, dadurch gekennzeichnet, daß die im spitzen Winkel zueinander stehenden Zuführungs- (5) und Abgabestutzen (6) und der Anschlußkonus (9) jeweils rechtwinklig zu ihrem Rohrteil (4 bzw. 4a) ausgebildet sind.

3. Zuführungsstück für die Atemgase nach Anspruch 1 und 2, dadurch gekennzeichnet, daß die Abdichtung zwischen den Teilen (1, 2, 3) über Dichtungsringe (16) erfolgt.

4. Zuführungsstück für die Atemgase nach Anspruch 1 bis 3, dadurch gekennzeichnet, daß das Patiententeil (2) dem Anschlußkonus (9) entgegengesetzt einen verschließbaren Stutzen (11) besitzt.

5. Zuführungsstück für die Atemgase nach Anspruch 1 bis 4, dadurch gekennzeichnet, daß der Anschlußkonus (9) einen Anschluß (12) für die Abnahme des Atemwegdruckes besitzt.

**Claims**

1. A feed piece for respiratory gas at highfrequency respiration, the feed piece having a supply connection (5), and a patient part (2) which carries a connecting cone (9), together with a flow part (3), positioned between the supply connection (5) and patient part (2), and having connecting nozzles for, and into which are directed, an injector nozzle (8) and an ejector nozzle (10), characterised in that a ring pipe part (1), with the supply connection (5) and a discharge connection (6) opening out into this on a tube part (4), and the patient part (2), on a corresponding tube part (4a), are, through snap-arms (14) on these tube parts (4, 4a), rotatably connected and held together by means of the flow part (3), and in that the injector nozzle (8) and the ejector nozzle (10) are arranged in each case in the end wall of the tube member (4) or (4a).

2. A feed piece for respiratory gas according to claim 1, characterised in that the supply- (5) and discharge-connections (6), at an acute angle in relation to each other, and the connecting cone (9) are in each case at right-angles to their tube part (4) or (4a).

3. A feed piece for respiratory gas according to claims 1 and 2, characterised in that the sealing between the parts (1, 2, 3) is effected by means of sealing rings (16).

4. A feed piece for respiratory gas according to claims 1 to 3, characterised in that the patient member (2) opposite the connecting cone (9) has a connecting piece (11) able to be locked

5. A feed piece for respiratory gas according to claims 1 to 4, characterised in that the connecting cone (9) has a connection (12) for decreasing the respiratory passage pressure.

**Revendications**

1. Elément d'amenée pour les gaz respiratoires destinés à une respiration artificielle haute fréquence, comprenant une tubulure d'amenée (5) et une partie (2) destinée au patient qui supporte un cône de raccordement (9), ainsi qu'une partie d'écoulement (3) disposée entre elles, avec des buses collectrices pour une buse d'injection (8) et une buse d'éjection (10) orientée dans la direction de cette partie, caractérisé en ce qu'une section de conduite annulaire (1) dans laquelle débouchent la tubulure d'amenée (5) et une tubulure de décharge (6), et la partie destinée au patient (2), sont reliées l'une à l'autre de façon rotative au moyen de la partie d'écoulement (3) en étant respectivement fixées à une partie tubulaire (4) et à une partie tubulaire correspondante (4a) et par l'intermédiaire de pattes d'encliquetage (14) prévues sur ces parties tubulaires (4, 4a), et en ce que la buse d'injection (8) et la buse d'éjection (10) sont respectivement montées dans la paroi frontale de la partie tubulaire correspondante (4 ou 4a).

2. Elément d'amenée pour des gaz respiratoires selon la revendication 1, caractérisée en ce que les tubulures d'amenée (5) et de décharge (6), qui forment un angle aigu entre elles, et le cône de raccordement (9) sont respectivement orientés à angle droit par rapport à leur partie tubulaire correspondante (4 ou 4a).

3. Elément d'amenée pour des gaz respiratoires selon les revendications 1 et 2, caractérisé en ce que l'étanchéité entre les parties (1, 2, 3) est réalisée au moyen de joints annulaires (16).

4. Elément d'amenée pour des gaz respiratoires selon l'une des revendications 1 à 3, caractérisé en ce que la partie (2) destinée au patient présente, à l'opposé du cône de raccordement (9), une tubulure (11) pouvant être obturée.

5. Elément d'amenée pour des gaz respiratoires selon l'une des revendications 1 à 4, caractérisé en ce que le cône de raccordement (9) comprend un raccord (12) pour le contrôle de pression dans les voies respiratoires.

Fig. 1

Fig. 2